(19)

Europäisches Patentamt

European Patent Office

Office européen des brevets

(11) **EP 4 032 549 A1**

(12) **EUROPEAN PATENT APPLICATION**

(43) Date of publication:
   **27.07.2022  Bulletin 2022/30**

(21) Application number: **21152805.4**

(22) Date of filing: **21.01.2021**

(51) International Patent Classification (IPC):
   **A61K 39/395** (2006.01)    **A61K 39/40** (2006.01)
   **C07K 16/00** (2006.01)     **A61P 7/00** (2006.01)
   **A61P 37/02** (2006.01)     **A61P 31/04** (2006.01)
   **A61P 39/02** (2006.01)

(52) Cooperative Patent Classification (CPC):
   **A61K 39/39516; A61K 39/40; C07K 16/00;**
   A61K 2039/507; A61K 2039/55; C07K 2317/76

(84) Designated Contracting States:
   **AL AT BE BG CH CY CZ DE DK EE ES FI FR GB
   GR HR HU IE IS IT LI LT LU LV MC MK MT NL NO
   PL PT RO RS SE SI SK SM TR**
   Designated Extension States:
   **BA ME**
   Designated Validation States:
   **KH MA MD TN**

(71) Applicant: **Octapharma AG**
   **8853 Lachen (CH)**

(72) Inventors:
   • **Walter, Olaf**
     **8738 Uetliburg (CH)**
   • **Römisch, Jürgen Roland**
     **2440 Gramatneudiedl (AT)**

(74) Representative: **Ullrich & Naumann PartG mbB**
   **Schneidmühlstrasse 21**
   **69115 Heidelberg (DE)**

(54) **PHARMACEUTICAL COMBINATION PRODUCT AND USES THEREOF**

(57)   The present invention relates to pharmaceutical products and in particular to pharmaceutical combination products. The invention further relates to uses of such products in medical treatment. Embodiments of the invention have been especially developed as pharmaceutical combination products of a human polyvalent immunoglobulin composition and one or more additional monoclonal antibodies and/or fragments thereof against one or more corresponding antigens of at least one pathogen and/or pathogen-derived toxin, in particular for use in the treatment of immunodeficiency disorders and/or co-morbidities, and will be described hereinafter with reference to this application. However, it will be appreciated that the invention is not limited to this particular field of use.

EP 4 032 549 A1

**Description**

**FIELD OF THE INVENTION**

The present invention relates to pharmaceutical products and in particular to pharmaceutical combination products. The invention further relates to uses of such products in medical treatment. Embodiments of the invention have been especially developed as pharmaceutical combination products of a human polyvalent immunoglobulin composition and one or more additional monoclonal antibodies and/or fragments thereof against one or more corresponding antigens of at least one pathogen and/or pathogen-derived toxin, in particular for use in the treatment of immunodeficiency disorders, and will be described hereinafter with reference to this application. However, it will be appreciated that the invention is not limited to this particular field of use.

**BACKGROUND OF THE INVENTION**

Any discussion of the background art throughout the specification should in no way be considered as an admission that such art is widely known or forms part of common general knowledge in the field

Human immunoglobulin compositions are typically prepared from blood plasma, which is collected and pooled from thousands of donors. Among the various forms of immunoglobulins within polyvalent immunoglobulin compositions, immunoglobulin G (IgG) typically constitutes the major component. Such compositions are known to be particularly useful in the treatment of a number of diseases and disorders, such as in the treatment of inflammatory and autoimmune diseases but also in the treatment of diseases that require antibody replacement therapy, such as, for example, primary immunodeficiencies (PID), secondary immunodeficiencies (SID) and hypogammaglobulinaemia. Human immunoglobulin compositions are also known to be useful for therapeutic immunomodulation in the treatment of diseases or disorders requiring the same, such as, for example, primary immune thrombocytopenia (ITP), Guillain Barre syndrome, Kawasaki disease, chronic inflammatory demyelinating polyneuropathy (CIDP) or multifocal motor neuropathy (MMN). Generally, patients requiring treatment with immunoglobulin compositions are immune-compromised patients.

Notwithstanding the above, immunoglobulin compositions obtained from pooled human plasma can also be prepared such that immunoglobulin M (IgM) constitutes the major component of the composition. Such IgM concentrates are useful in the treatment of specific indications (often distinct to those for which IgG concentrates are useful) such as, for example, severe Community Acquired Pneumonia (sCAP).

A human immunoglobulin composition obtained from pooled human plasma contains a large number of different antibody populations, wherein each population is specific to a certain antigen and the overall composition provides antibodies against most pathogens and pathogen-derived toxins to which the general population is typically exposed to in the geographical region in which the plasma donations were collected, i.e. the composition is reflective of the overall antibody repertoire present in the general population. While pooling plasma donations for the preparation of human immunoglobulin compositions beneficially increases the degree of polyvalency of such a composition, it also dilutes (i.e. reduces the concentration of) individual, beneficial antibodies within such compositions. This dilution effect is also reflected in the long time required for human immunoglobulin compositions obtained from pooled plasma donations to acquire protective antibody titres against new pathogens, for example new pathogens to which the general donor population is only exposed during the course of an epidemic or pandemic.

In this regard, it is noteworthy that for regulatory approval the quality - and thereby the effectiveness - of human polyvalent immunoglobulin compositions is determined by comparing the titres of antigen-specific antibodies in the composition or by comparing the neutralization capacity of the composition conveyed by such antibodies against certain pathogens prevalent in the general population with that of an established standard of the respective regulatory authority (for example against polio virus, measles virus or diphtheria toxin). The minimal titres are thought necessary to ensure that a patient receiving the immunoglobulin composition receives a certain minimum amount of protection against these prevalent pathogens. However, as has been observed over the last decades, for example with respect to anti-measles antibody titres, plasma donations from individuals, who contracted the measles virus at some point in their life, have higher antibody titres against than those of individuals who acquired measles immunity by vaccination. Given that vaccination against measles is prescribed in most immunization schedules around the world, the anti-measles antibody titre of human immunoglobulin preparations has been steadily decreasing over time as the proportion of plasma donors, who still underwent a measles infection, steadily decreases. In the case of measles, the regulatory authorities have responded in lowering the minimal measles antibody titres in line with what is still readily achievable in human immunoglobulin preparations from pooled plasma, while still providing a protective effect. However, uncertainty abounds whether lower titres of vaccination-induced antibodies against a pathogen or pathogen-derived toxin will ultimately suffice to allow continuously providing human immunoglobulin preparations from pooled plasma, which provide the necessary protection against otherwise prevalent pathogens, in the future. This is of course of particular concern for immune-compromised patients.

One approach to obtain human immunoglobulin compositions with a higher antibody titre against a pathogen is to pre-select the plasma donor population such that only plasma from donors with high antibody titres against the pathogen (e.g. plasma donors, who in their previous life underwent infection) is pooled to obtain a so-called hyperimmune immunoglobulin composition. However, the large amount of donors typically required in the commercial preparation of human immunoglobulin and the difficulty of successfully identifying suitable donors combined with the simply low numbers of such suitable donors, render the preparation of hyperimmune immunoglobulin concentrates produced from (pooled) plasma cumbersome and the resulting compositions expensive and even rare. This approach does not seem suitable to serve the need of the general patient population dependent on human immunoglobulin compositions.

In light of the above, there is a need in the art for improved human immunoglobulin compositions, which provide a recipient patient with the degree of protection against a pathogen above that provided by an immunoglobulin preparation based on pooled plasma donations from the general donor population.

It is an object of the present invention to overcome or ameliorate at least one of the disadvantages of the prior art, or to provide a useful alternative. In particular, it is an object of the present invention to provide immunoglobulin compositions providing a patient with an improved and tailored antibody composition when faced with a specific infectious challenge.

## SUMMARY OF THE INVENTION

The present invention provides a solution to the above-formulated problem by enhancing existing human polyvalent immunoglobulin compositions with one or more additional monoclonal antibodies or fragments thereof against one or more corresponding antigens of at least one specific pathogen and/or pathogen-derived toxin such as to provide a pharmaceutical combination product:

(a) which provides a patient receiving the combination product with protection against pathogens or pathogen-derived toxins, which are not yet sufficiently prevalent in the general population such that a conventional human polyvalent immunoglobulin composition yields protective antibody titres; and, additionally or alternatively,

(b) which has increased pathogen-specific antibody titres to supplement and/or restore antibody titres against antigens of prevalent pathogens or pathogen-derived toxins known to be waning in conventional human polyvalent immunoglobulin compositions.

Accordingly, in a first aspect, the present invention relates to a pharmaceutical combination product comprising:

(a) a human polyvalent immunoglobulin composition; and

(b) one or more additional monoclonal antibodies or fragments thereof against one or more corresponding antigens of at least one pathogen and/or pathogen-derived toxin,

wherein the human polyvalent immunoglobulin composition is previously obtained from pooled human plasma, and wherein, through the one or more additional monoclonal antibodies and/or fragments thereof, the combination product has an enhanced capability to protect a patient receiving the combination product from a disease caused by the at least one pathogen and/or pathogen-derived toxin compared to the human polyvalent immunoglobulin composition alone.

As such, in embodiments where the combination product comprises more than one, i.e. several, different monoclonal antibodies or fragments thereof, these are directed against the same number of different antigens, which correspond with, i.e. are bound by, the respective monoclonal antibody or fragment. Furthermore, in some embodiments, these several different antigens may be different antigens of the same pathogen or pathogen-derived toxin or of several different pathogens and/or pathogen toxins.

Typically, the human polyvalent immunoglobulin composition alone has no titre or only a low titre of antibodies against the one or more antigens of the at least one pathogen and/or pathogen-derived toxin, and/or the one or more additional monoclonal antibodies and/or fragments thereof supplement antibodies against one or more antigens of the at least one pathogen and/or pathogen-derived toxin in the human polyvalent immunoglobulin composition.

In particular, the one or more additional monoclonal antibodies and/or fragments thereof are capable of neutralizing the at least one pathogen and/or pathogen-derived toxin such that the combination product has an increased neutralization capacity against the pathogen and/or pathogen-derived toxin compared to the human polyvalent immunoglobulin composition alone. Typically, given the high specificity of the one or more neutralizing additional monoclonal antibodies and/or fragments thereof capable of neutralizing the at least pathogen and/or pathogen-derived toxin, only a small amount of monoclonal antibody or fragment thereof must be spiked into an existing human polyvalent immunoglobulin composition such as to significantly enhance the combination product's neutralization capacity.

Importantly, the pharmaceutical combination product according to the first aspect is particularly suitable for use in medical prophylaxis and/or treatment of a disease in a patient caused by the at least one pathogen and/or pathogen-derived toxin.

Accordingly, in further aspects, the present invention also relates to a method of medical prophylaxis and/or treatment of a disease caused by at least one pathogen and/or pathogen-derived toxin comprising the antigen or antigens against which the one or more additional monoclonal antibodies and/or fragments thereof comprised in the combination product are directed, wherein the method comprises administering the pharmaceutical combination product of the first aspect to a patient in need thereof.

Similarly, in a further aspect, the present invention also relates to use of the pharmaceutical combination product of the first aspect in the manufacture of a medicament for medical prophylaxis and/or treatment, such as for the treatment of a disease caused by at least one pathogen and/or pathogen-derived toxin, which comprises the one or more corresponding antigens against which the one or more additional monoclonal antibodies and/or fragments thereof are directed.

In all of the above aspects, when used in such treatment, the pharmaceutical combination product provides the patient receiving the combination product with an enhanced protection from a disease caused by the at least one pathogen and/or pathogen-derived toxin compared to the human polyvalent immunoglobulin composition alone.

In yet a further aspect, the present invention relates to a process for the production of a pharmaceutical combination product according to the first aspect, comprising adding one or more additional monoclonal antibodies or fragments thereof against one or more corresponding antigens of at least one pathogen and/or pathogen-derived toxin to a human polyvalent immunoglobulin composition, wherein the human polyvalent immunoglobulin composition is previously obtained from pooled human plasma.

## DETAILED DESCRIPTION OF THE INVENTION

In order to provide a clear and consistent understanding of the specification and claims, and the scope to be given such terms, the following definitions are provided.

## Definitions

The term **"pharmaceutical combination product"** in the context of this disclosure includes a product in which a single composition comprises both active ingredients, i.e. both (a) a human polyvalent immunoglobulin composition and (b) one or more additional monoclonal antibodies or fragments thereof against one or more corresponding antigens of at least one pathogen and/or pathogen-derived toxin. However, the term also includes a product, such as a kit, in which the two active ingredients (a) a human polyvalent immunoglobulin composition and (b) one or more additional monoclonal antibodies or fragments thereof against one or more corresponding antigens of at least one pathogen and/or pathogen-derived toxin are provided in separate compositions, possibly even for simultaneous or sequential administration via the same or a different route of administration. For example, a pharmaceutical combination product in the context of the present disclosure may comprise a formulation of (a) the human polyvalent immunoglobulin composition for subcutaneous administration, and a formulation of (b) one or more additional monoclonal antibodies or fragments thereof against one or more corresponding antigens of at least one pathogen or pathogen-derived toxin adapted for intravenous administration. Advantageously, however, when the pharmaceutical combination product of the present invention is provided as a kit of parts, a sufficient amount of the one or more additional monoclonal antibodies and/or fragments thereof can be provided such that the practitioner administering the product can still adjust the final titre administered in accordance with the individual patient's needs.

In the context or the present invention, the term "monoclonal antibody" refers to an antibody that has been made by cloning its coding sequence from an individual and unique white blood cell, preferably from a B-Cell. Various platforms for the generation of monoclonal antibodies are known.

For example, antibody phage display is a well-validated tool to generate recombinant antibodies against toxins for diagnostic and therapeutic purposes (see Wenzel, E, et al. "Human antibodies neutralizing diphtheria toxin in vitro and in vivo." Scientific reports vol. 10,1 571. 17 Jan. 2020; Miethe S, et al. "Development of Germline-Humanized Antibodies Neutralizing Botulinum Neurotoxin A and B." PLoS One. 2016;11:e0161446; Pelat T, et al. "Isolation of a human-like antibody fragment (scFv) that neutralizes ricin biological activity." BMC Biotechnol. 2009;9:60; the respective disclosures regarding this methodology are incorporated herein). An overview of antibodies generated by phage display against pathogens and toxins is given by Kuhn et al. ("Recombinant antibodies for diagnostics and therapy against pathogens and toxins generated by phage display." Proteom. Clin. Appl. 2016;10:922-948).

Other tools for the generation of monoclonal antibodies use single cell emulsion droplet microfluidics technology to capture and recreate the entire antibody repertoire as recombinant antibody libraries from which the most potent antibodies against at least one pathogen and/or pathogen-derived toxin can be identified and produced (see Michael A, et al. "Antibody repertoire analysis of mouse immunization protocols using microfluidics and molecular genomics" mAbs, vol. 11:5, 870-883, the respective disclosures of which are incorporated herein). Such technology platforms are particularly potent to identify and - subsequently - recombinantly produce highly effective human antibodies against a new pathogen or pathogen-derived toxin, for example, from white blood cell donors, who have only recently overcome an infection by the pathogen.

A **"fragment"** of a monoclonal antibody in the present disclosure encompasses a single-chain variable fragment (scFv) or an antigen-binding fragment (Fab - obtained through papain cleavage of an IgG; F(ab') - obtained through pepsin cleavage of an IgG; F(ab')2 - obtained pepsin cleavage of an IgG and subsequent β-mercaptoethanol treatment; and/or nanobody - antibody fragment consisting of a single monomeric antigen-specific variable antibody domain).

A **"humanized antibody"** or **"humanized monoclonal antibody"** in the context of the present disclosure refers to an antibody from a non-human species (such as mice, rats and/or guinea pigs), which has been modified such as to more closely match a human antibody, to reduce an immune reaction when the antibody is administered to a human patient. This typically entails genetically engineering the non-human antibody in questions such as to modify its protein sequence to allow for human-like secondary modification patterns of the antibody molecule such as, for example and without limitation, glycosylation- and/or sialylation-pattern etc.). While chimeric antibodies are not necessarily humanized antibodies, in the context of the present disclosure, the terms **"humanized antibody"** or **"humanized monoclonal antibody"** also encompass chimeric antibodies engineered such as to combine an antigen-specific protein sequence or fragment of a non-human antibody (such as, for example and without limitation, a mouse antibody Fab-fragment) with a human antibody sequence or fragment (such as, for example and without limitation, an fragment crystallisable (Fc) domain of an IgG) such as to provide an antibody largely constituted of human protein sequences.

In the context of the present invention, the term **"pathogen"** refers to a biological agent that causes disease or illness to its host through disruption of the host's normal physiology. Pathogens in the sense of the present invention are, in particular, pathogens relevant to human and/or animal health and include viral, bacterial and fungal pathogens. Pathogens with relevance for human and/or animal health include, without limitation: Cytomegalo virus, Entero virus, Epstein-Barr virus, Hepatitis A virus, Hepatitis B virus, Measles virus, Parvovirus, Polio virus, Rubella virus, Coronavirus, Varicella zoster, *Bordetella pertussis, Haemophilus influenza, Klebsiella pneumonia, Streptococcus pneumonia, Streptococcus pyogenes, Moraxella catarrhalis, Mycoplasma pneumonia, Neisseria gonorrhoea, Neisseria meningitides, Pseudomonas* and *Staphylococcus.*

In the context of the present invention, the term **"pathogen-derived toxin"** refers to a substance harmful to humans and/or animals, which is produced by a pathogen. Such pathogen-derived toxins include small molecules, peptides or proteins produced by a pathogen that are capable of causing disease or illness to a human or animal through the toxin's interaction with biological macromolecules (such as enzymes or cellular receptors) to disrupt normal human or animal physiology. Pathogen-derived toxins with relevance for human and/or animal health include, without limitation:

- diphtheria toxin - an exotoxin produced and secreted by *Corynebacterium;*
- streptolysin O - an immunogenic, oxygen-labile streptococcal hemolytic exotoxin produced by most strains of group A and many strains of groups C and G *Streptococcus* bacteria; and
- tetanus toxin (also known as tetanospasmin, spasmogenic toxin or TeNT) - *a* potent neurotoxin produced by the vegetative cell of *Clostridium tetani* in anaerobic conditions, causing *tetanus.*

In addition to the above definitions, and unless the context clearly requires otherwise, throughout the description and the claims, the words "comprise", "comprising", and the like are to be construed in an inclusive sense as opposed to an exclusive or exhaustive sense; that is to say, in the sense of "including, but not limited to".

Further, reference throughout this specification to **"one embodiment", "some embodiments"** or **"an embodiment"** means that a particular feature, structure or characteristic described in connection with the embodiment is included in at least one embodiment of the present invention. Thus, appearances of the phrases "in one embodiment", "in some embodiments" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

As used herein, unless otherwise specified the use of the ordinal adjectives **"first", "second", "third",** etc., to describe a common object, merely indicate that different instances of like objects are being referred to, and are not intended to imply that the objects so described must be in a given sequence, either temporally, spatially, in ranking, or in any other manner.

As used herein, the term **"exemplary"** is used in the sense of providing examples, as opposed to indicating quality. That is, an **"exemplary embodiment"** is an embodiment provided as an example, as opposed to necessarily being an embodiment of exemplary quality.

## Products and methods of the invention

As indicated above, the present invention provides a solution to the above-formulated problem by enhancing existing human polyvalent immunoglobulin compositions with one or more additional monoclonal antibodies against one or more corresponding antigens of at least one specific pathogen and/or pathogen-derived toxin.

As such, the invention relates to a pharmaceutical combination product comprising:

(a) a human polyvalent immunoglobulin composition; and
(b) one or more additional monoclonal antibodies or fragments thereof against one or more corresponding antigens of at least one pathogen and/or pathogen-derived toxin,

wherein the human polyvalent immunoglobulin composition is previously obtained from pooled human plasma, and
wherein, through the one or more additional monoclonal antibodies and/or fragments thereof, the combination product has an enhanced capability to protect a patient receiving the combination product from a disease caused by the at least one pathogen and/or pathogen-derived toxin compared to the human polyvalent immunoglobulin composition alone.

The **"capability"** to protect a patient receiving the combination product from a disease caused by the at least one pathogen and/or pathogen-derived toxin depends on the amount of antibodies against the at least one pathogen and/or pathogen-derived toxin that is delivered to the patient in a certain mg immunoglobulin per/kg dose of the combination product.

To establish and ensure this capability, the combination product must comprise an amount of antibodies against one or more antigens of the at least one pathogen and/or pathogen-derived toxin sufficient to achieve a degree of neutralization comparable to that of an established reference standard for a given time period. Typically, this time period is the time period between the administration of a one mg immunoglobulin per/kg dose of the combination product and a subsequent further mg immunoglobulin per/kg dose thereof, wherein the respective antibody titre of the established reference standard is known to convey protection against the particular pathogen or pathogen-derived toxin. Typically, the regulatory authorities provide the relevant reference standard. For example, one of the relevant established reference standards against which the neutralization capacity of a human polyvalent immunoglobulin composition against measles virus or poliovirus is compared is the Center for Biologics Evaluation and Research (CBER) Standard lot 176 (referred to herein also as "NIH 176"). A relevant established reference standard, against which the neutralization capacity of a human polyvalent immunoglobulin composition against diphtheria toxin is compared too, is the standardized unit/mL neutralization activity of Diphtheria Anti-Toxin (DAT). This amount, in turn, is considered necessary for the patient to maintain a serum antibody titre against the pathogen or pathogen-derived toxin.

In the pharmaceutical combination product of the present invention, this capability is enhanced compared to the corresponding capability of the human polyvalent immunoglobulin composition is obtained from pooled human plasma alone through the presence of the one or more additional monoclonal antibodies and/or fragments thereof.

The term **"enhanced"** in this context means:

- that the combination product, through the one or more additional monoclonal antibodies and/or fragments thereof, has gained a *de novo* capability to protect a patient receiving the combination product from a disease caused by the at least one pathogen and/or pathogen-derived toxin, compared to the human polyvalent immunoglobulin composition alone, i.e. such that the combination product, upon administration to a patient, can protect the patient receiving the combination product from a disease caused by the at least one pathogen and/or pathogen-derived toxin, whereas the human polyvalent immunoglobulin composition alone cannot; or
- that the one or more additional monoclonal antibodies and/or fragments thereof supplement antibodies against one or more antigens of the at least one pathogen and/or pathogen-derived toxin already present in the human polyvalent immunoglobulin composition, i.e. such that the combination product, upon administration to a patient, can protect the patient receiving the combination product better or more efficiently from a disease caused by the at least one pathogen and/or pathogen-derived toxin compared to the human polyvalent immunoglobulin composition alone.

In both instances, the combination product of the present invention can be seen as an enhanced or improved human polyvalent immunoglobulin composition for administration to a patient in need thereof for treating a disease caused by the at least one pathogen and/or pathogen-derived toxin.

Accordingly, in some embodiments of the invention, the human polyvalent immunoglobulin composition alone has no titre or only a low titre of antibodies against one or more antigens of the at least one pathogen and/or pathogen-derived toxin.

In accordance with the above, a human polyvalent immunoglobulin composition, which alone, i.e. without any additional antibodies or fragments thereof against one or more corresponding antigens of the at least one pathogen and/or pathogen-derived toxin, has **"no titre or only a low titre"** of antibodies against one or more antigens of the pathogen or pathogen-derived toxin, refers to a human polyvalent immunoglobulin composition, which has a either no protective capability against the at least one pathogen and/or pathogen-derived toxin at all or a lesser protective capability against the at least one pathogen and/or pathogen-derived toxin compared to the pharmaceutical combination product of the present invention.

In some embodiments, the one or more additional monoclonal antibodies and/or fragments thereof supplement antibodies against the one or more corresponding antigens of the at least one pathogen and/or pathogen-derived toxin in the human polyvalent immunoglobulin composition. As such, in embodiments where the combination product comprises several different monoclonal antibodies or fragments thereof, these are directed against several different antigens corresponding with the respective antibody or fragment. Furthermore, in some embodiments, these several different antigens may be antigens of the same at least one pathogen or pathogen-derived toxin or of several different pathogens and/or pathogen toxins.

Accordingly, the combination product may, for example, comprise between 1 and 15, preferably between 1 and 10 or between 1 and 5 or between 5 and 15 or between 5 and 10 or between 3 and 12, different monoclonal antibodies and/or fragments thereof. For example, the combination product may comprise 2, 3, 4, 5, 6, 7, 8, or 10 different monoclonal antibodies and/or fragments thereof.

Notwithstanding the above, the total amount of the one or more additional monoclonal antibodies or fragments thereof against the one or more corresponding antigens (referred to in the below expression by *"mAB1", "mAB2",* etc.) of the one or more pathogens or pathogen-derived toxins (referred to in the below expression by *"Pat1", "Pat2"* etc.) in the combination product is less than, and therefore does not exceed, 1 % w/w of the total amount of immunoglobulins in the combination product. In particular, this relationship may be expressed as follows:

$$\sum\nolimits_{(mAb)} = \sum\nolimits_{(mAbPat1)} + \sum\nolimits_{(mAbPat2)} + \sum\nolimits_{(mAbPat2)} + \dots$$

and/or

$$\sum\nolimits_{(mAbPat1)} = \sum\nolimits_{(mAb1Pat1)} + \sum\nolimits_{(mAb2Pat1)} + \sum\nolimits_{(mAb3Pat1)} + \dots$$

In particular embodiments, In a preferred embodiment, each of the monoclonal antibodies or fragments thereof against the one or more corresponding antigens are present at a concentration of at least 1 pM or at least 5 pM or at least 10 pM or at least 50 pM or at least 100 pM, and, typically, the total amount of all additional monoclonal antibodies or fragments thereof does not exceed 5% w/w, preferably it does not exceed 4% w/w or 3% w/w or 2% w/w or 1% w/w, of the total amount of immunoglobulin of the combination product.

As described above, the supplementing of antibodies in the human polyvalent immunoglobulin composition, i.e. the supplementing of an already existing antibody titre, is achieved through the one or more additional monoclonal antibodies and/or fragments thereof against one or more corresponding antigens of specifically targeted pathogens or pathogen-derived toxins. In embodiments where the one or more additional monoclonal antibodies and/or fragments thereof are capable of neutralizing the at least one pathogen and/or pathogen-derived toxin and supplement neutralizing antibodies against the at least one pathogen and/or pathogen-derived toxin already present in the human polyvalent immunoglobulin composition, the pharmaceutical combination product consequently has an $IC_{50}$ for neutralization of the at least one pathogen and/or pathogen-derived toxin that is lower than that of human polyvalent immunoglobulin composition alone.

As indicated above, the required minimum specification/level for measles neutralizing antibody levels in human polyvalent immunoglobulin compositions have been decreased incrementally to accommodate decreasing antibody titres in the general plasma donor population. In fact, the adjustment of the minimum measles antibody specification was, in part, prompted by the FDA's observation that increasing numbers of immunoglobulin product lots from different manufacturers failed to meet the measles antibody specification and by the concern that, if that trend continued, it could lead to a product shortage. As such, the minimum measles antibody specification required for human polyvalent immunoglobulin compositions in the US was reduced from 0.60 times that of the NIH 176 in 2008 to 0.48 times that of NIH 176 and again from 0.48 times that of the NIH 176 in 2018 to the current specification of 0.36 times that of the NIH 176.

According to Chen RT, et al. "Measles antibody: reevaluation of protective titres." (J Infect Dis 1990; 162: 1036-42), the protective serum level for immunocompetent individuals is estimated at 120 mIU/mL. While the protective antibody level for immune-compromised patients is not known, in 2018 (see FDA's "Letter to Immune Globulin (Human) Licensed Manufacturers: Option to Lower Lot Release Specification for Required Measles Antibody Potency Testing" dated November 5, 2018; freely available at https://www.fda.gov/media/118428/download) the FDA proposed double that concentration, namely 240 mIU/mL for measles antibodies in human polyvalent immunoglobulin compositions. Based on pharmacokinetic (PK) modelling, the FDA considers that a 400 mg/kg dose of a human polyvalent immunoglobulin composition administered intravenously and comprising a measles antibody titre of 0.36 x NIH 176, provides a patient with anti-measles antibody levels of at least 240 mIU/mL for up to two weeks.

In the FDA's PK modelling a number of assumptions were necessary namely that:

- the estimated titre of NIH 176 is 42 IU/mL (16.5% solution;
- the equilibration of an intravenously administered human polyvalent immunoglobulin composition between intravascular and extravascular compartments occurs 5 days after infusion;
- at equilibrium, 40% of intravenously administered human polyvalent immunoglobulin composition is distributed intravascularly, and 60% extravascularly.
- the half-life of intravenously administered human polyvalent immunoglobulin composition is approximately 22 days.

*FDA's calculation for post-infusion PK parameters, for 400 mg/kg IGIV at 0.36 x NIH 176:*

- Specific activity of lot 176 = 42 IU-mL/165 mg-mL = 0.25 IU/mg
- 0.25 IU/mg x 0.36 = 0.09 IU/mg
- 400 mg/kg dose x 0.09 IU/mg = 36 IU/kg dose in human
- Serum titre just after infusion (Cmax) = 36 IU/kg x kg/40 mL blood = 0.9 IU/mL, or 900 mIU/mL.

Serum titre after equilibration (5 days) = 900 mIU/mL x 0.4 [40% of IG will be intravascular] = 360 mIU/mL.
Serum titre at 13.5 days = 270 mIU/mL, if dose is 400 mg/kg.
Serum titre at 22 days (t1/2) = 360/2 = 180 mIU/mL, if dose is 400 mg/kg, or = 238.5, if dose is 530 mg/kg.

The measles antibody titre can be determined as neutralization titre $NT_{50}$ [1:X], i.e. the reciprocal dilution resulting in 50% measles virus neutralization, by a fully validated neutralization assay on Vero cells (ATCC CCL-81), preferably in duplicate, essentially as described in Modrof, J, et al. "Measles virus neutralizing antibodies in intravenous immunoglobulins: is an increase by revaccination of plasma donors possible?" (J Infect Dis, 216 (2017), pp. 977-980; the relevant disclosures of which are incorporated by reference here).

Similar, observations regarding a decrease in the neutralization capacity of human polyvalent immunoglobulin compositions have also been reported for other pathogens for which immunity is predominantly achieved through vaccination, rather than through overcoming an actual infection.

Accordingly, the benefit of the pharmaceutical combination product, especially for immune-compromised patients will be appreciated given that the neutralizing antibody titre against one or more relevant pathogens and/or pathogen-derived toxins of the combination product can be easily adjusted to meet patient needs - in particular to levels of above the FDA's required minimum specifications for the respective pathogens and/or pathogen-derived toxins, will be appreciated.

Therefore, in some embodiments, the one or more additional monoclonal antibodies and/or fragments thereof are capable of neutralizing the at least one pathogen and/or pathogen-derived toxin such that the combination product has an increased neutralization capacity against the at least one pathogen and/or pathogen-derived toxin compared to the human polyvalent immunoglobulin composition alone. Preferably, the neutralization capacity of the combination product is increased by at least 2 fold, or at least 3 fold, or at least 5 fold, or at least 7 fold, or at least 10 fold, or at least 20 fold, or at least 30 fold, or at least 40 fold, or at least 50 fold, or at least 100 fold, or at least 250 fold, or at least 500 fold, or at least 750 fold, or at least 1000 fold.

In the context of the present invention, an antibody that is **"capable of neutralizing at least one pathogen and/or pathogen-derived toxin"** refers to a **"neutralizing antibody** (NAb)"** and both terms may be used interchangeably throughout the present disclosure. An antibody's capability to neutralize at least one pathogen and/or pathogen-derived toxin renders the respective pathogen non-infective and/or non-pathogenic.

For example, and without limitation, a neutralizing antibody might bind specifically to an antigen of a surface structure of an infectious particle (such as to the spike protein of a virus) and may, thereby, either entirely block the virus from interacting with a human host cell, which it might otherwise infect, or, alternatively, while still allowing for some degree of interaction, only block the virus from infecting the host cell. With respect to a neutralizing antibody against a pathogen-derived toxin, the antibody specifically binds to a certain antigen of the toxin, which is otherwise important for the toxin's effect on a host cell's metabolism. For example, and without limitation, with respect to an antibody capable of neutralizing diphtheria toxin, the antibody might specifically bind to an antigen of the toxin such that, when bound by the antibody, the toxin can no longer interact with the heparin-binding epidermal growth factor precursor HB-EGFP, thereby rendering the toxin ineffective.

Neutralization assays are capable of being performed and measured in different ways, including the use of techniques such as plaque reduction (which compares counts of virus plaques in control wells with those in inoculated cultures), microneutralization and which is performed in microtitre plates filled with small amounts of sera), and colorimetric assays (which depend on biomarkers indicating metabolic inhibition of the virus; see Kaslow, RA, et al. "Viral Infections of Humans: Epidemiology and Control", (5th ed.). Springer. p. 56. ISBN 9781489974488, the relevant disclosures of which are incorporated by reference here).

In some embodiments, the pharmaceutical combination product according to the invention comprises the human polyvalent immunoglobulin composition and the one or more additional monoclonal antibodies and/or fragments thereof in a single composition.

Advantageously, the amount of the one or more additional monoclonal antibodies and/or fragments thereof can be adjusted, for example to increase specific antibody titre in a given mg immunoglobulin per/kg dose of the combination product to be administered, such that higher serum titres of the relevant antibodies and/or fragments thereof can be achieved and allow for longer time periods between individual instances of administration without compromising the patient's protection or to simply provide higher specific protection against a pathogen or pathogen-specific toxin than achievable with standard human polyvalent immunoglobulin compositions obtained from pooled plasma. For example when administered to patients that have already been exposed to the pathogen, i.e. in an acute treatment scenario.

In some embodiments, the one or more additional monoclonal antibodies and/or fragments thereof are human or humanized monoclonal antibodies and/or fragments thereof. Preferably, the one or more additional monoclonal antibodies and/or fragments thereof alone have an $IC_{50}$ for neutralization of the pathogen or pathogen-derived toxin of below 1 nM, preferably below 500 pM or below 250 pM or below 100 pM or below 50 pM.

In particular embodiments, the one or more corresponding antigens are selected from:

- viral antigens, preferably Coronaviridae-, Flaviviridae-, Herpesviridae-, Matonaviridae-, Paramyxoviridae-, Picornaviridae-, Polyomaviridae-, Pneumoviridae- or Reoviridae-antigens;
- bacterial antigens, preferably *Alcaligenaceae-, Campylobacteraceae-, Clostridiaceae-, Corynebacteriaceae-, Enterobacteriaceae-, Enterococcaceae-, Helicobacteraceae-, Moraxellaceae-, Mycoplasmataceae-, Neisseriaceae-, Pasteurellaceae-, Pseudomonadaceae-, Streptococcaceae-* or *Staphylococcaceae-antigens;* and
- fungal antigens, preferably *Candida-, Aspergillus-, Cryptococcus-, Histo-plasma-, Pneumocytis-* or *Stachybotrys-antigens.*

In particular, embodiments, the one or more corresponding antigens are one or more antigens of:

- Cytomegalo virus;
- Entero virus;
- Epstein-Barr virus;
- Hepatitis A virus;
- Hepatitis B virus;
- Measles virus;
- Parvovirus B19;
- Poliovirus type 1, 2 or 3;
- Rubella virus;
- SARS-Cov-2;
- Varicella zoster virus;
- *Bordetella pertussis;*
- *Haemophilus influenzae;*
- *Klebsiella pneumonia;*
- *Streptococcus pneumonia;*
- *Streptococcus pyogenes;*
- *Moraxella catarrhalis;*
- *Mycoplasma pneumoniae;*
- *Neisseria gonorrhoea;*
- *Neisseria meningitidis;*
- *Pseudomonas,* preferably *Pseudomonas aeruginosa,*
- *Staphylococcus,* preferably *Staphylococcus aureus;*
- Diphtheria toxin;
- Streptolysin O; and/or
- Tetanus Toxin.

The pharmaceutical combination product in accordance with the invention, wherein the one or more additional monoclonal antibodies and/or fragments thereof are monoclonal antibodies and/or fragments thereof against one or more corresponding antigens of the measles virus, one or more corresponding antigens of the poliovirus and/or one or more corresponding antigens of the diphtheria toxin. Specifically, in some embodiments, the combination product has:

- a neutralizing antibody titre against said measles virus of at least 0.01 x NIH 176, preferably at least 0.05 x NIH 176 or at least 0.1 x NIH 176 or at least 0.36 x NIH 176 or at least 0.48 x NIH 176 or at least 0.60; and/or

- a neutralizing antibody titre against said diphtheria toxin equivalent to that of at least 0.1 units/mL of diphtheria antitoxin (DAT), preferably equivalent to that of at least 0.5 units/mL of DAT or of at least 1.00 units/mL of DAT or of at least 1.50 units/mL of DAT or of at least 1.75 units/mL of DAT or of at least 2.00 units/mL of DAT or of at least 2.50 units/mL of DAT or of at least 3.00 units/mL of DAT or of at least 5.00 units/mL of DAT or of at least 10.00 units/mL of DAT or of at least 20.00 units/mL of DAT or of at least 30.00 units/mL of DAT; and/or

- a neutralizing antibody titre against said polio virus types 1, 2 and/or 3 of at least 0.01 x NIH 176, preferably at least 0.08 x NIH 176 or at least 0.17 x NIH 176 or at least 0.25 x NIH 176 or at least 0.4 x NIH 176 or at least 0.5 x NIH 176 or at least 0.75 x NIH 176; and/or

- a neutralizing antibody titre against said hepatitis A virus of at least 1 IU/ml, preferably at least 3 IU/ml or at least 5 IU/ml or at least 10 IU/ml or at least 20 IU/ml or at least 30 IU/ml or at least 40 IU/ml or at least 50 IU/ml.

Alternatively or additionally, in some embodiments the one or more antigens of the at least one pathogen and/or pathogen-derived toxin are antigens of a multi-resistant pathogen, preferably of a multi-resistant bacterial pathogen such as Methicillin-resistant *Staphylococcus aureus* (MRSA) and wherein the one or more additional monoclonal antibodies and/or fragments thereof are human monoclonal antibodies capable of neutralizing the multi-resistant pathogen.

It will be appreciated that, while the present invention is described with reference to monoclonal antibodies or fragments thereof against one or more corresponding antigens of pathogens or pathogen-derived toxins, the pharmaceutical combination product of the present invention can also comprise monoclonal antibodies against one or more corresponding clinically-relevant tumour-associated antigens (e.g. against MUC-1), against one or more corresponding clinically-relevant allergy associated antigens (e.g. against IgE) or against one or more corresponding clinically-relevant self-antigens (e.g. an anti-idiotypic antibody against Factor VIII auto-antibodies).

As indicated above, only a small fraction of the one or more additional neutralizing monoclonal antibodies and/or fragments thereof is required to be spiked into an existing human polyvalent immunoglobulin composition to significantly enhance the combination product's capability to protect a patient receiving it from a disease caused by the at least one pathogen and/or pathogen-derived toxin, compared to the human polyvalent immunoglobulin composition alone.

As also already indicated above, the pharmaceutical combination product according to the invention is particularly suitable for use in medical prophylaxis and/or treatment of a disease in a patient caused by the at least one pathogen and/or pathogen-derived toxin.

Typically, the use comprises administering the pharmaceutical composition to a patient having been exposed to a viral, bacterial or fungal pathogen or pathogen-derived toxin and/or to a patient suffering from an acute viral, bacterial or fungal infection, wherein, optionally, the patient is an immune-compromised patient, such as a patient suffering from a primary immunodeficiency (PID), from a secondary immunodeficiency (SID), from hypogammaglobulinaemia, from primary immune thrombocytopenia (ITP), from Guillain Barre syndrome, from Kawasaki disease, from chronic inflammatory demyelinating polyneuropathy (CIDP) or from multifocal motor neuropathy (MMN).

The most recent classifications of primary immunodeficiencies (PID) by the International Union of Immunological Societies (IUIS) Expert Committee on Primary Immunodeficiency is described detail in Al-Herz W et al., "Primary immunodeficiency diseases: an update on the classification from the International Union of Immunological Societies Expert Committee for Primary Immunodeficiency", Frontiers in Immunology 2014 (freely available at https://primaryimmune.org/healthcare-professionals/idf-healthcare-professional-publications; Tables 1 through 9 of Al-Herz W *et al.*, listing the known PIDs are herein incorporated by reference).

In some embodiments, the use comprises administering the human polyvalent immunoglobulin composition and the one or more additional monoclonal antibodies and/or fragments thereof simultaneously. As such, even if combination product is a kit in which both active ingredients are provided separately, these are administered to a patient in need thereof simultaneously.

In some embodiments, the use comprises administering the pharmaceutical combination product to the patient via intravenous, intramuscular or subcutaneous administration.

In addition to the above-described pharmaceutical combination products, the present invention also relates to a process for the production of the above-described pharmaceutical combination product, comprising adding one or more additional monoclonal antibodies or fragments thereof against one or more corresponding antigens of at least one pathogen and/or pathogen-derived toxin to a human polyvalent immunoglobulin composition, wherein the human polyvalent immunoglobulin composition is previously obtained from pooled human plasma.

In some embodiments, the production process further comprises the steps of:

- initially identifying one or more monoclonal antibodies or fragments thereof from a collection of human antibodies, preferably wherein each identified monoclonal antibody is encoded by a single B-cell, such as a unique human B-cells, and
- recombinantly producing the identified monoclonal antibodies or fragments thereof in a human cell line expression system for addition to the human polyvalent immunoglobulin composition as the one or more additional monoclonal antibodies or fragments thereof.

In some embodiments, the process may instead comprise the steps of

- initially identifying one or more monoclonal antibodies or fragments thereof from a collection of non-human antibodies;
- modifying the protein sequences of the identified by recombinant engineering such as generate suitable antigen-specific fragments thereof or humanized antibodies; and
- recombinantly producing the antigen-specific fragments or the humanized antibodies in a human cell line expression system for addition to the human polyvalent immunoglobulin composition as the one or more additional monoclonal antibodies or fragments thereof.

Typically, the one or more additional monoclonal antibodies or fragments thereof are initially identified based on their capability to neutralize the at least one pathogen and/or pathogen-derived toxin.

Many modifications and other embodiments of the invention set forth herein will come to mind to the one skilled in the art to which the invention pertains having the benefit of the teachings presented in the foregoing description and the associated drawings. Therefore, it is to be understood that the invention is not to be limited to the specific embodiments disclosed and that modifications and other embodiments are intended to be included within the scope of the appended claims. Although specific terms are employed herein, they are used in a generic and descriptive sense only and not for purposes of limitation.

**Claims**

1. A pharmaceutical combination product comprising:

   (a) a human polyvalent immunoglobulin composition; and
   (b) one or more additional monoclonal antibodies or fragments thereof against one or more corresponding antigens of at least one pathogen or pathogen-derived toxin,

   wherein said human polyvalent immunoglobulin composition is previously obtained from pooled human plasma, and wherein, through said one or more additional monoclonal antibodies and/or fragments thereof, said combination product has an enhanced capability to protect a patient receiving said combination product from a disease caused by said at least one pathogen and/or pathogen-derived toxin compared to the human polyvalent immunoglobulin composition alone.

2. The pharmaceutical combination product according to claim 1, wherein said human polyvalent immunoglobulin composition alone has no titre or only a low titre of antibodies against one or more antigens of said at least one pathogen or pathogen-derived toxin.

3. The pharmaceutical combination product according to claim 1 or claim 2, wherein said one or more additional monoclonal antibodies or fragments thereof supplement antibodies against one or more antigens of said at least one pathogen or pathogen-derived toxin in the human polyvalent immunoglobulin composition.

4. The pharmaceutical combination product according to any one of claims 1 to 3, wherein said one or more additional monoclonal antibodies or fragments thereof are capable of neutralizing said at least one pathogen or pathogen-derived toxin such that said combination product has an increased neutralization capacity against said at least one pathogen or pathogen-derived toxin compared to the human polyvalent immunoglobulin composition alone.

5. The pharmaceutical combination product according to claim 4, wherein said neutralization capacity of said combination product is increased by at least 5 fold, or at least 10 fold, or at least 50 fold, or at least 100 fold.

6. The pharmaceutical combination product according to any one of the preceding claims, wherein said combination product comprises said human polyvalent immunoglobulin composition and said one or more additional monoclonal antibodies or fragments thereof in a single composition, wherein, optionally, said one or more additional monoclonal antibodies or fragments thereof are directed against one or more antigens of more pathogens and/or pathogen-derived toxins.

7. The pharmaceutical combination product according to any one of the preceding claims, wherein said one or more additional monoclonal antibodies or fragments thereof are human or humanized monoclonal antibodies or fragments thereof.

8. The pharmaceutical combination product according to claim 7, wherein each of said one or more additional monoclonal antibodies or fragments thereof alone has an $IC_{50}$ for neutralization of said at least one pathogen or pathogen-derived toxin of below 1000 nM, preferably below 100 nM, more preferably below 50 nM.

9. The pharmaceutical combination product according to any one of the preceding claims, wherein said one or more corresponding antigens are selected from:

   - viral antigens, preferably Coronaviridae- , Flaviviridae-, Herpesviridae-, Matonaviridae-, Paramyxoviridae-, Picornaviridae-, Polyomaviridae-, Pneumoviridae- and/or Reoviridae-antigens;
   - bacterial antigens, preferably Alcaligenaceae-, Campylobacteraceae-, Clostridiaceae-, Corynebacteriaceae-, Enterobacteriaceae-, Enterococcaceae-, Helicobacteraceae-, Moraxellaceae-, Mycoplasmataceae-, Neisseriaceae-, Pasteurellaceae-, Pseudomonadaceae-, Streptococcaceae- and/or Staphylococcaceae-antigens; and
   - fungal antigens, preferably Candida-, Aspergillus-, Cryptococcus-, Histoplasma-, Pneumocytis- and/or Stachybotrys-antigens.

10. The pharmaceutical combination product according to any one of the preceding claims, wherein said one or more corresponding antigens are one or more antigens of:

    - Cytomegalo virus;
    - Entero virus;
    - Epstein-Barr virus;
    - Hepatitis A virus;
    - Hepatitis B virus;
    - Measles virus;
    - Parvovirus B19;
    - Poliovirus types 1, 2 or 3;
    - Rubella virus;
    - SARS-Cov-2;
    - Varicella zoster virus;
    - *Bordetella pertussis;*
    - *Haemophilus influenzae;*
    - *Klebsiella pneumonia;*
    - *Streptococcus pneumonia;*
    - *Streptococcus pyogenes;*
    - *Moraxella catarrhalis;*
    - *Mycoplasma pneumoniae;*
    - *Neisseria gonorrhoea;*
    - *Neisseria meningitidis;*
    - *Pseudomonas;*
    - *Staphylococcus;*
    - Diphtheria toxin;
    - Streptolysin O; and/or
    - Tetanus Toxin.

11. The pharmaceutical combination product according to claim 10, wherein said one or more additional monoclonal antibodies or fragments thereof are monoclonal antibodies or fragments thereof against one or more antigens of said measles virus, said poliovirus, said hepatitis A virus and/or said diphtheria toxin

12. The pharmaceutical combination product according to claim 11, wherein said combination product has:

- a neutralizing antibody titre against said measles virus of at least 0.1 x NIH 176, preferably of at least 0.36 x NIH 176 or at least 0.48 x NIH 176 or at least 0.60; and/or

- a neutralizing antibody titre against said diphtheria toxin equivalent to that of at least 2.00 units/mL of DAT, preferably of at least 5.00 units/mL of DAT or of at least 10.00 units/mL of DAT; and/or

- a neutralizing antibody titre against said polio virus types 1, 2 and/or 3 of at least 0.08 x NIH 176, preferably of at least 0.50 x NIH 176 or at least 0.75 x NIH 176; and/or

- a neutralizing antibody titre against said hepatitis A virus of at least 10 IU/ml, preferably at least 20 IU/ml or at least 40 IU/ml.

13. The pharmaceutical combination product according to claim 10, wherein each of the monoclonal antibodies or fragments thereof against said one or more corresponding antigens is present at a concentration of at least 1 pM, preferably of at least 10 pM or at least 100 pM, and wherein the total amount of all additional monoclonal antibodies or fragments thereof does not exceed 5% w/w of the total amount of immunoglobulin of the combination product.

14. The pharmaceutical combination product according to any one of claims 1 to 9, wherein said one or more corresponding antigens are one or more antigens of a multi-resistant pathogen, preferably of a multi-resistant bacterial pathogen such as Methicillin-resistant Staphylococcus aureus (MRSA) and wherein said one or more additional monoclonal antibodies or fragments thereof are human monoclonal antibodies or fragments thereof capable of neutralizing said multi-resistant pathogen.

15. The pharmaceutical combination product according to any one of the preceding claims for use in medical prophylaxis and/or treatment of a disease in a patient caused by said pathogen or pathogen-derived toxin.

16. The pharmaceutical combination product for use according to claim 15, wherein said use comprises administering the pharmaceutical composition to a patient having been exposed to viral, bacterial or fungal pathogen or pathogen-derived toxin and/or to a patient suffering from an acute viral, bacterial or fungal infection, wherein, optionally, said patient is an immune-compromised patient, such as a patient suffering from a primary immunodeficiency (PID), from a secondary immunodeficiency (SID), from hypogammaglobulinaemia, from primary immune thrombocytopenia (ITP), from Guillain Barre syndrome, from Kawasaki disease, from chronic inflammatory demyelinating polyneuropathy (CIDP) or from multifocal motor neuropathy (MMN).

17. A process for the production of a pharmaceutical combination product according to any one of the preceding claims, comprising adding one or more additional monoclonal antibodies or fragments thereof against one or more corresponding antigens of at least one pathogen or pathogen-derived toxin to a human polyvalent immunoglobulin composition, wherein said human polyvalent immunoglobulin composition is previously obtained from pooled human plasma.

18. The process of claim 17, further comprising the steps of:

   - initially identifying one or more monoclonal antibodies or fragments thereof from a collection of human antibodies, preferably wherein each identified monoclonal antibody is encoded by a single B-cell, such as a unique human B-cells, and
   - recombinantly producing said identified monoclonal antibodies or fragments thereof in a human cell line expression system for addition to said human polyvalent immunoglobulin composition as said one or more additional monoclonal antibodies or fragments thereof.

19. The process of claim 17, further comprising the steps of:

   - initially identifying one or more monoclonal antibodies or fragments thereof from a collection of non-human antibodies;
   - modifying the protein sequences of said identified by recombinant engineering such as generate suitable antigen-specific fragments thereof or humanized antibodies; and
   - recombinantly producing said antigen-specific fragments or said humanized antibodies in a human cell line expression system for addition to said human polyvalent immunoglobulin composition as said one or more additional monoclonal antibodies or fragments thereof.

Europäisches
Patentamt

European
Patent Office

Office européen
des brevets

EUROPEAN SEARCH REPORT

Application Number

EP 21 15 2805

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/175605 A1 (LIVERPOOL SCHOOL TROPICAL MEDICINE [GB]) 19 September 2019 (2019-09-19) * The whole document, in particular p.40, para.1 * | 1-19 | INV. A61K39/395 A61K39/40 C07K16/00 A61P7/00 A61P37/02 A61P31/04 A61P39/02 |
| X | J P DEVINCENZO ET AL: "Respiratory syncytial virus immune globulin treatment of lower respiratory tract infection in pediatric patients undergoing bone marrow transplantation - a compassionate use experience", BONE MARROW TRANSPLANTATION, vol. 25, no. 2, 1 January 2000 (2000-01-01), pages 161-165, XP055263769, DOI: 10.1038/sj.bmt.1702118 * The whole document, in particular, p.164, col.2. * | 1-19 | |
| X | PEREZ ELENA E ET AL: "Update on the use of immunoglobulin in human disease: A review of evidence", JOURNAL OF ALLERGY AND CLINICAL IMMUNOLOGY, ELSEVIER, AMSTERDAM, NL, vol. 139, no. 3, 29 December 2016 (2016-12-29), XP029915883, ISSN: 0091-6749, DOI: 10.1016/J.JACI.2016.09.023 * The whole document, in particular, p.S18, 'pneumonia and pneumonitis' describing CMV-IGIV * | 1-19 | TECHNICAL FIELDS SEARCHED (IPC) A61K C07K A61P |

-/--

The present search report has been drawn up for all claims

| Place of search | Date of completion of the search | Examiner |
|---|---|---|
| Munich | 7 July 2021 | Chapman, Rob |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 21 15 2805

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DAVID A LOEFFLER: "Should development of Alzheimer's disease-specific intravenous immunoglobulin be considered?", JOURNAL OF NEUROINFLAMMATION, BIOMED CENTRAL LTD., LONDON, GB, vol. 11, no. 1, 5 December 2014 (2014-12-05), page 198, XP021206209, ISSN: 1742-2094, DOI: 10.1186/S12974-014-0198-Z * The whole document, in particular, the abstract * | 1-19 | |
| X | STROHL WILLIAM R ET AL: "13 - Multiple antibody and multi-specificity approaches", THERAPEUTIC ANTIBODY ENGINEERING, WOODHEAD PUBLISHING LIMITED, GB, PAGE(S) 299 - 328, 1 January 2012 (2012-01-01), XP009528503, ISBN: 978-1-907568-37-4 Retrieved from the Internet: URL:https://doi.org/10.1533/9781908818096.299 * The whole document, in particular, section 13.4 * | 1-19 | TECHNICAL FIELDS SEARCHED (IPC) |
| A | HARTUNG H -P ET AL: "Clinical applications of intravenous immunoglobulins (IVIg) - beyond immunodeficiencies and neurology", CLINICAL AND EXPERIMENTAL IMMUNOLOGY, vol. 158, no. Suppl. 1, December 2009 (2009-12), pages 23-33, XP55821509, ISSN: 0009-9104 * the whole document * | 1-19 | |

The present search report has been drawn up for all claims

| Place of search | Date of completion of the search | Examiner |
|---|---|---|
| Munich | 7 July 2021 | Chapman, Rob |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 2 of 2

## ANNEX TO THE EUROPEAN SEARCH REPORT
## ON EUROPEAN PATENT APPLICATION NO.

EP 21 15 2805

This annex lists the patent family members relating to the patent documents cited in the above-mentioned European search report.
The members are as contained in the European Patent Office EDP file on
The European Patent Office is in no way liable for these particulars which are merely given for the purpose of information.

07-07-2021

| Patent document cited in search report | | | Publication date | Patent family member(s) | | | Publication date |
|---|---|---|---|---|---|---|---|
| WO 2019175605 | A1 | | 19-09-2019 | AU | 2019235513 | A1 | 08-10-2020 |
| | | | | CN | 112236449 | A | 15-01-2021 |
| | | | | EP | 3765492 | A1 | 20-01-2021 |
| | | | | US | 2021024615 | A1 | 28-01-2021 |
| | | | | WO | 2019175605 | A1 | 19-09-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WENZEL, E et al.** Human antibodies neutralizing diphtheria toxin in vitro and in vivo. *Scientific reports,* 17 January 2020, vol. 10, 1-571
- **MIETHE S et al.** Development of Germline-Humanized Antibodies Neutralizing Botulinum Neurotoxin A and B. *PLoS One,* 2016, vol. 11, e0161446
- **PELAT T et al.** Isolation of a human-like antibody fragment (scFv) that neutralizes ricin biological activity. *BMC Biotechnol.,* 2009, vol. 9, 60
- **KUHN et al.** Recombinant antibodies for diagnostics and therapy against pathogens and toxins generated by phage display. *Proteom. Clin. Appl.,* 2016, vol. 10, 922-948
- **MICHAEL A et al.** Antibody repertoire analysis of mouse immunization protocols using microfluidics and molecular genomics. *mAbs,* vol. 11 (5), 870-883
- **CHEN RT et al.** Measles antibody: reevaluation of protective titres. *J Infect Dis,* 1990, vol. 162, 1036-42

- **FDA'S.** Letter to Immune Globulin (Human) Licensed Manufacturers: Option to Lower Lot Release Specification for Required Measles Antibody Potency Testing. 05 November 2018
- **MODROF, J et al.** Measles virus neutralizing antibodies in intravenous immunoglobulins: is an increase by revaccination of plasma donors possible?. *J Infect Dis,* 2017, vol. 216, 977-980
- **KASLOW, RA et al.** Viral Infections of Humans: Epidemiology and Control. Springer, 56
- **AL-HERZ W et al.** Primary immunodeficiency diseases: an update on the classification from the International Union of Immunological Societies Expert Committee for Primary Immunodeficiency. *Frontiers in Immunology,* 2014, https://primaryimmune.org/health-care-professionals/idf-healthcare-professional-publications